# EUROPÄISCHE PATENTANMELDUNG

(11) **EP 2 730 285 A1**
(43) Veröffentlichungstag der Anmeldung: **14.05.2014**
(21) Anmeldenummer: 12191622.5
(22) Anmeldetag: 07.11.2012
(51) Int. Cl.: A61K 31/7084, A61P 11/06, A61P 11/08

(54) **Verwendung von NAD und beta-NAD als Bronchodilatator**

(71) Anmelder: Justus-Liebig-Universität Gießen, 35390 Gießen (DE)
(72) Erfinder: Kummer, Wolfgang, 35075 Gladenbach (DE); Jurastow, Innokentij Gennadiwitsch, 35440 Großen-Linden (DE); Krasteva-Christ, Gabriela, 35390 Gießen (DE)
(74) Vertreter: Stumpf, Peter

(57) **Zusammenfassung**

Das Molekül NAD sowie dessen Diastereomer β-NAD haben eine muskelrelaxierende Wirkung auf die Muskulatur der Atemwege, insbesondere auf die Bronchialmuskulatur. Damit eröffnen NAD sowie dessen Diastereomer β-NAD einen neuen Ansatz zur Therapie und Prophylaxe von zahlreichen Erkrankungen, Störungen und Zuständen, bei denen die Konzentration an freiem Kalzium in den Muskelzellen der Atemwegsmuskulatur krankhaft verändert ist.

Daher ist eine geeignete pharmazeutische Zubereitung mit dem Wirkstoff NAD oder dessen Diastereomer β-NAD als Medikament gegen Erkrankungen vorgesehen, die mit einer Konstriktion der Atemwegsmuskulatur einhergehen.

## Beschreibung

### Definitionen und Abkürzungen

**Atemwegsmuskulatur:** Muskulatur der Atemwege; Trachealmuskulatur, Bronchialmuskulatur
**ATP:** Adenosintriphosphat
β**-NAD:** entspricht beta-NAD, β-Nicotinamidadenindinukleotid, β-NAD⁺, β-Nicotinsäureamid-Adenin-Dinukleotid, β-Nadid, β-NADH und β-NADH₂; beta-NAD ist ein Diastereomer des NAD
**Bronchodilatation:** Erweiterung der Luftwege innerhalb der Lungen, damit einhergehend eine funktionelle Erniedrigung des Atemwegswiderstandes
**Bronchokonstriktion:** Verengung der Luftwege innerhalb der Lungen, damit einhergehend eine funktionelle Erhöhung des Atemwegswiderstandes **Bronchospasmus:** Krampfartige Bronchokonstriktion
**cGMP:** cyclic Guanosine Monophosphate, zyklisches Guanosinmonophosphat **COPD:** Chronic Obstructive Pulmonary Disease, chronisch obstruktive Lungenerkrankung
**CTX:** Choleratoxin; Exotoxin der Bakterien *Vibrio cholerae* und *Vibrio eltor* **Dilatation, Relaxation; dilatativ, relaxierend:** Erschlaffung der Muskelzellen und damit der Muskulatur; muskelerschlaffend
**GPCR:** G-Protein-gekoppelte Rezeptoren; Rezeptoren in der Zellmembran, die Signale über GTP-bindende Proteine in das Zellinnere weiterleiten
**GTP:** Guanosintriphosphat
**HEPES:** 2-(4-(2-Hydroxyethyl)-1-piperazinyl)-ethansulfonsäure; Puffersubstanz, beispielsweise für Zellkulturmedien
**KM:** Körpermasse des Patienten
**Kontraktion:** Anspannung der Muskelzellen und damit der Muskulatur
**L-NAME:** L-N^{G}-Nitroarginin-Methyl-Ester
**MEM, MEM Medium:** Minimal Essential Medium; Zellkulturmedium
**MRS2179:** N6methyl-2'-deoxyadenosin-3'5'-bisphosphate; Selektiver Antagonist des P2Y₁-Rezeptors
**NAD:** entspricht Nicotinamidadenindinukleotid, NAD⁺, Nicotinsäureamid-Adenin-Dinukleotid, Nadid, NADH und NADH₂; NAD ist ein bekanntes körpereigenes Molekül
**NOS:** Stickstoffmonoxyd-Synthase
**PDE4:** 3',5'-zyklische Nukleotid-Phosphodiesterase aus der vierten Familie
**PKA:** Protein-Kinase A; Enzym, welches den Transfer einer Phosphatgruppe katalysiert
**PPADS:** Pyridoxalphosphate-6-azophenyl-2',4'-disulfonic acid
**PTX:** Pertussistoxin; Exotoxin des Bakteriums *Bordetella pertussis*
**P2Y:** Rezeptor des Botenstoffs Adenosintriphosphat
**Trachea:** Luftröhre

### Stand der Technik

Das bekannte körpereigene Molekül NAD sowie dessen Diastereomer β-NAD fungieren als Hydridionen-übertragende Koenzyme, die an zahlreichen Redoxreaktionen des Stoffwechsels der Zelle beteiligt sind, beispielsweise als energieliefernde Koenzyme der Atmungskette.

Bei Erkrankungen, die mit einer Einengung der Atemwege, zum Beispiel einer Bronchokonstriktion oder einem Bronchospasmus einhergehen, wird dieses schwerwiegende Symptom regelmäßig durch einen Krampf der Atemwegs- und Bronchialmuskulatur oder eine Entzündung der Atemwege verursacht. Solche Erkrankungen sind beispielsweise Asthma jedweden Typs oder COPD. Die durch die Bronchokonstriktion verursachte Atemnot stellt für den Patienten eine sehr starke Belastung dar und kann bis zu Erstickungsanfällen mit Todesfolge führen. Zur Behandlung der durch Asthma jedweden Typs verursachten Bronchokonstriktion stehen verschiedene Medikamente zur Verfügung, die sich in die zwei Gruppen Entzündungshemmer und Bronchodilatatoren einteilen lassen. Häufig werden auch Medikamente aus den beiden Gruppen kombiniert. Alle bekannten Medikamente zur Behandlung der durch Asthma jedweden Typs verursachten Bronchokonstriktion sind bei den beiden Krankheitsbildern unterschiedlich wirksam und können oftmals keine Beschwerdefreiheit bewirken. Zusätzlich können bei längerer oder dauerhafter Anwendung schwerwiegende Nebenwirkungen auftreten, beispielsweise beim Einsatz von Kortikosteroiden. Eine ursächliche Therapie, die die Ursachen für die Entstehung der Bronchokonstriktion beseitigt, ist nicht bekannt.

Für die Behandlung der COPD stehen derzeit Medikamente aus zwei Wirkstoffgruppen zur Verfügung: Einerseits muskarinische Rezeptorantagonisten, welche die Wirkung des körpereigenen Bronchokonstriktors Azetylcholin hemmen, andererseits Beta-Agonisten, welche aktiv eine Erweiterung der Bronchien stimulieren. Allerdings ist damit nur eine symptomatische Therapie möglich, wobei der Therapieerfolg bei einzelnen Patienten sehr unterschiedlich sein kann.

Es besteht also ein großer Bedarf an einem Medikament zur Behandlung der durch Asthma jedweden Typs oder COPD verursachten Bronchokonstriktion, welches ein vorteilhaftes Wirkungs-Nebenwirkungsverhältnis aufweist.

### Aufgabe

Die Aufgabe der vorliegenden Erfindung ist es, ein Mittel bereit zu stellen, das als Medikament für Menschen und Tiere, bevorzugt Säuger, bei allen Erkrankungen, Störungen und Zuständen, bei denen eine Konstriktion der Atemwegsmuskulatur vorliegt, eingesetzt wird. Dieses Mittel soll eine zuverlässige muskelrelaxierende Wirkung auf die Atemwegsmuskulatur haben. Insbesondere soll die Bronchokonstriktion bei den Erkrankungen Asthma und COPD behandelt werden.

### Lösung der Aufgabe

Diese Aufgabe wird erfindungsgemäß gelöst durch ein Mittel gemäß Anspruch 1. Auf die in den Unteransprüchen angegebenen Ausführungsmöglichkeiten wird Bezug genommen.

Vorzugsweise umfasst das Mittel NAD (Nicotinamidadenosindinukleotid) oder β-NAD oder ein pharmazeutisch annehmbares Salz, Diastereomer, Stereoisomer, Tautomer, Solvat, Polymorph, isotopische Variation davon zur Herstellung eines Medikamentes zur Behandlung und Prophylaxe von Menschen und Tieren. Alternativ umfasst das Mittel zusätzlich pharmazeutisch unschädliche Exzipientien und/oder Additive. Alternativ umfasst das Mittel zusätzlich andere therapeutische Mittel aus der Gruppe der Entzündungshemmer, Bronchodilatatoren, muskarinischen Rezeptorantagonisten oder Beta-Agonisten.

Vorzugsweise wird das Mittel verwendet:
1. zur Herstellung eines Medikamentes zur Behandlung und Prophylaxe von Erkrankungen, Störungen und Zuständen bei Menschen und Tieren, bei denen die Konzentration an freiem Kalzium in den Muskelzellen der Atemwegsmuskulatur krankhaft verändert ist.
2. zur Herstellung eines Medikamentes zur Behandlung und Prophylaxe von Erkrankungen, Störungen und Zuständen bei Menschen und Tieren, bei denen eine Konstriktion der Atemwegsmuskulatur vorliegt.
3. zur Herstellung eines Medikamentes zur Behandlung und Prophylaxe von Asthma oder chronisch obstruktiver Lungenerkrankungen (COPD).

Vorzugsweise wird das erfindungsgemäße Mittel verabreicht als Spray, Aerosol, Trockenpulver oder Liposomen.

Vorzugsweise wird das erfindungsgemäße Mittel inhalativ verabreicht.

Überraschenderweise wurde festgestellt, dass NAD sowie dessen Diastereomer β-NAD oder ein Stereoisomer, Tautomer, Solvat, Polymorph, eine isotopische Variation davon eine muskelrelaxierende Wirkung auf die Muskulatur der Atemwege von Menschen und Tiere, bevorzugt Säugern hat, insbesondere auf die Bronchialmuskulatur. Damit sind NAD sowie dessen Diastereomer β-NAD, sowie pharmazeutisch akzeptable Zubereitungen mit NAD sowie dessen Diastereomer β-NAD zur Herstellung von Medikamenten gegen Erkrankungen geeignet, die mit einer Konstriktion der Atemwegsmuskulatur einhergehen.

Eigene wissenschaftliche Ergebnisse zeigen, dass die muskelrelaxierende Wirkung des NAD sowie dessen Diastereomers β-NAD auf die glatte Muskulatur der Atemwege erfolgt, diese Wirkung aber nicht identisch ist mit der Wirkung auf purinerge Rezeptoren der P2Y-Klasse, wie es bei der Darmmuskulatur beschrieben ist.

Die muskelrelaxierende Wirkung des NAD sowie dessen Diastereomers β-NAD auf die Muskulatur der Atemwege wird nachweislich nicht über die Entstehung von Stickstoffmonoxid verhindert.

Die muskelrelaxierende Wirkung des NAD sowie dessen Diastereomers β-NAD auf die Muskulatur der Atemwege wird nachweislich nicht von G-Protein gekoppelten Rezeptoren verhindert.

Die muskelrelaxierende Wirkung des NAD sowie dessen Diastereomers β-NAD auf die Muskulatur der Atemwege wird nachweislich nicht durch die Inhibition von PDE4 bewirkt.

Wahrscheinlich beruht der Wirkmechanismus des NAD und seines Diastereomers β-NAD auf einer Änderung der Konzentration an freiem Kalzium in den Muskelzellen der Atemwegsmuskulatur, welche zu einer Relaxation der Muskelzellen der Atemwegsmuskulatur führt.

Die in Anspruch 1 genannten Mittel NAD sowie dessen Diastereomer β-NAD oder ein Stereoisomer, Tautomer, Solvat, Polymorph, eine isotopische Variation davon werden zur Herstellung eines Medikamentes gegen Erkrankungen eingesetzt, die mit einer Konstriktion der Atemwegsmuskulatur einhergehen. Die Verabreichung der in Anspruch 1 genannten Mittel kann nach Wahl des behandelnden Arztes erfolgen. Die in Anspruch 1 genannten Mittel können per se, in Gemischen miteinander oder in Form von pharmazeutischen Präparationen, die eine enterale oder parenterale Verabreichung ermöglichen, verabreicht werden.

Vorteilhaft ist die Verabreichung der in Anspruch 1 genannten Mittel mittels inhalierbarer Darreichungsformen, damit eine Applikation des Mittels direkt an den Wirkort erreicht wird. Inhalierbare Darreichungsformen können Sprays, Aerosole, Trockenpulverinhalatoren oder in inhalierbare Liposomen verpackte Mittel sein. Die in Anspruch 1 genannten Mittel NAD sowie dessen Diastereomer β-NAD oder ein Stereoisomer, Tautomer, Solvat, Polymorph, eine isotopische Variation davon werden auch als ihre pharmazeutisch annehmbaren Salze, Diastereomere, Stereoisomere, Tautomere, Solvate, Polymorphe, isotopische Variationen, Gemische oder Zusammensetzungen davon verwendet.

Die in Anspruch 1 genannten Mittel NAD sowie dessen Diastereomer β-NAD oder ein Stereoisomer, Tautomer, Solvat, Polymorph, eine isotopische Variation davon sind für die Therapie und Prophylaxe von zahlreichen Erkrankungen, Störungen und Zuständen geeignet, bei denen die Konzentration an freiem Kalzium in den Muskelzellen der Atemwegsmuskulatur krankhaft verändert ist.

Insbesondere sind die in Anspruch 1 genannten Mittel NAD sowie dessen Diastereomer β-NAD oder ein Stereoisomer, Tautomer, Solvat, Polymorph, eine isotopische Variation davon sind für die Therapie und Prophylaxe von zahlreichen Erkrankungen, Störungen und Zuständen geeignet, bei denen eine Konstriktion der Atemwegsmuskulatur vorliegt, wie beispielsweise bei Asthma oder chronisch obstruktiver Lungenerkrankungen (COPD).

Die in Anspruch 1 genannten Mittel NAD sowie dessen Diastereomer β-NAD oder ein Stereoisomer, Tautomer, Solvat, Polymorph, eine isotopische Variation davon werden gemäß der Erfindung an Tiere, vorzugsweise an Säuger, und insbesondere an Menschen als Pharmazeutika zur Therapie oder Prophylaxe von zahlreichen Erkrankungen, Störungen und Zuständen verabreicht, bei denen eine Konstriktion der Atemwegsmuskulatur vorliegt, wie beispielsweise bei Asthma oder chronisch obstruktiver Lungenerkrankungen (COPD).

Für jede Darreichungsform wird der tägliche Dosierungsbereich bei menschlichen Patienten der Mittel nach Anspruch 1 üblicherweise 0,001 mg/kg KM bis 100 mg/kg KM sein.

Die in Anspruch 1 genannten Mittel können auch als Kombination mit einem oder mehreren weiteren therapeutischen Stoffen eingesetzt werden, beispielsweise mit Entzündungshemmern, Bronchodilatatoren, muskarinische Rezeptorantagonisten oder Beta-Agonisten, um das gewünschte therapeutische Endresultat einer ungestörten Atmung zu erzielen.

### Beispiele einer technischen Ausführung

### 1. Organbadversuche an der isolierten Trachea der Maus

Nach tierschutzgerechter Tötung einer Maus wurde die Trachea entnommen. Für die Experimente wurde der Luftröhrenabschnitt zwischen der fünften und zehnten Knorpelspange verwendet, da sich dort die Wandverhältnisse (geringerer Knorpelanteil) für Untersuchungen der Kontraktilität der Atemwege am besten eignen. Explantierte Luftröhrenabschnitte wurden in ein geeignetes Zellkulturmedium (z.B. MEM Medium) überführt. Das Organbad (Radnoti LLC, Monrovia, USA) bestand aus einem 15 ml fassenden doppelwandigem Glaszylinder, in dessen innerer Kammer die Trachea an Häkchen aufgespannt wurde. Die Temperatur wurde konstant auf 37° C gehalten, indem die äußere Kammer ständig mit 37° C warmem Wasser durchspült wurde. Die innere Kammer wurde durch Durchsprudeln mit einem geeigneten Gasgemisch (21% O₂ und 79% N₂) oxygeniert.

Die durch das Innere der Trachea gezogenen Häkchen waren an Kraftaufnehmern (PowerLAb 8/30; ADInstruments, Bella Vista, Australien) gekoppelt, wodurch die Kontraktionskraft der Trachealmuskulatur gemessen, aufgezeichnet und ausgewertet werden konnte (Labchart, ADInstruments, Bella Vista, Australien).

In die innere Kammer wurden die folgenden Substanzen in unterschiedlichen Konzentrationen zu dem Zellkulturmedium gegeben:
Muskarin 10 µM (Sigma Aldrich)
β-NAD 10 µM-5 mM (Sigma Aldrich)
L-N^{G}-Nitroarginin-Methyl-Ester (L-NAME) 1 mM (Sigma Aldrich)
Pertussistoxin (PTX) 2 µg/ml (Santa Cruz)
Choleratoxin (CTX) 2 µg/ml (Sigma Aldrich)
Suramin 30 µM (Sigma Aldrich)
MRS2179 30 µM (Sigma Aldrich)
Pyridoxalphosphate-6-azophenyl-2',4'-disulfonic acid (PPADS) 3 µM (Sigma Aldrich)
Adenosintriphosphat (ATP) 100 µM (Sigma Aldrich)
Adenosine-3',5'-cyclic monophosphorothioate, Rp-Isomer (Rp-cAMP) 100 µM (Sigma Aldrich)

Nach dem Aufhängen der Tracheaabschnitte wurde einige Minuten unter normalen Bedingungen (nicht stimuliert) der Basiswert der Trachealwandspannung ermittelt. Hierbei befindet sich die Trachealmuskulatur in einem entspannten Zustand.

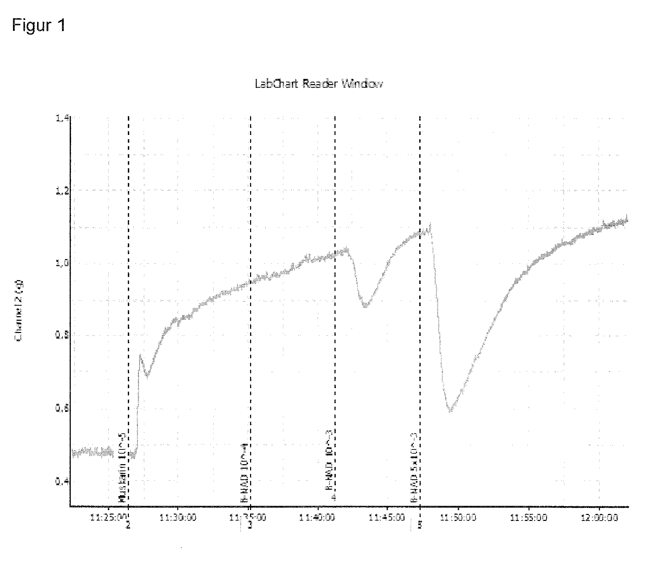

Dann wurde Muskarin bei jedem Versuch zugegeben, um die Trachealmuskulatur in den maximal kontrahierten Zustand zu bringen. Dieses ist in den folgenden Figuren als Anstieg in den Kurven zu sehen. Bei den ersten Versuchen wurde 15 Minuten nach der Kontraktion β-NAD in verschiedenen Konzentrationen zugegeben, um die Anhängigkeit der Antwort von der Substanzmenge zu untersuchen. Es wurde eine Erweiterung (Dilatation) der Trachea verzeichnet, die abhängig von der Menge des β-NAD war. Die Dilatation sieht man in der folgenden Figur 1 als Abfall im Kurvenverlauf. In der höchsten eingesetzten Konzentration (5x10⁻³ M) wurde die durch Muskarin hervorgerufene maximale Kontraktion um 75-80% rückgängig gemacht (Figur 1). Als β-NAD-Effekt wird die muskelrelaxierende Wirkung des NAD und des β-NAD auf die glatten Muskelzellen (d.h. die glatte Muskulatur) der Atemwegsmuskulatur verstanden.

### 2. Überprüfung des (NO)-cGMP-Signalweges

Typischerweise erfolgt die Dilatation der glatten Muskelzellen und damit der glatten Muskulatur, wie beispielsweise der Muskulatur des Magen-Darm-Traktes oder der Atemwegsmuskulatur, durch den Stickstoffmonoxyd-cGMP-Signalweg (NOcGMP pathway), bei dem eine NO-Synthase (NOS), von der es mehrere Isotypen gibt, die Aminosäure Arginin in Citrullin und den Dilatator NO umwandelt (2 L-Arginin + 3 NADPH + 4 O₂ → 2 L-Citrullin + 4 H₂O + 2 NO). Der Stickstoffmonoxyd-cGMP-Signalweg führt durch die Aktivierung der löslichen Guanylatzyklase zur Bildung von cGMP und damit über weitere Zwischenschritte zu einer Dilatation glatter Muskelzellen.

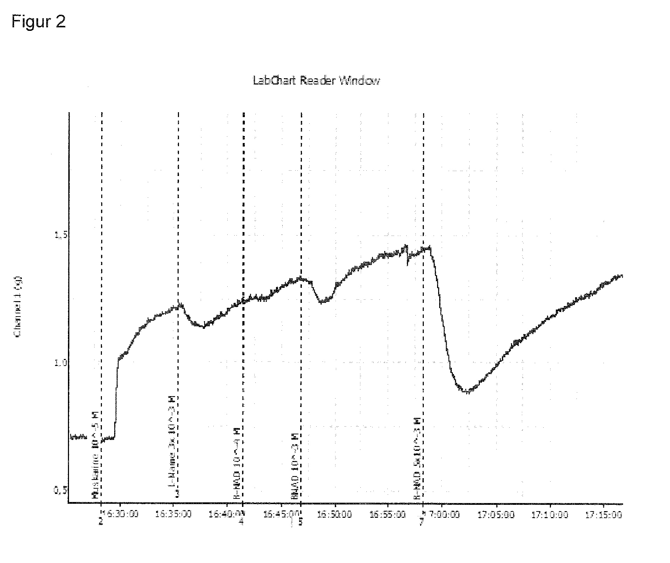

Um diesen Signalweg zu unterbinden, wurde der NOS-Inhibitor L-NAME eingesetzt. Dies führte aber nicht zu einer Aufhebung des β-NAD-Effektes, das daher unabhängig von diesem Signalweg operiert (Figur 2).

### 3. Überprüfung der Wirkung von β-NAD auf purinerge Rezeptoren der P2Y-Reihe

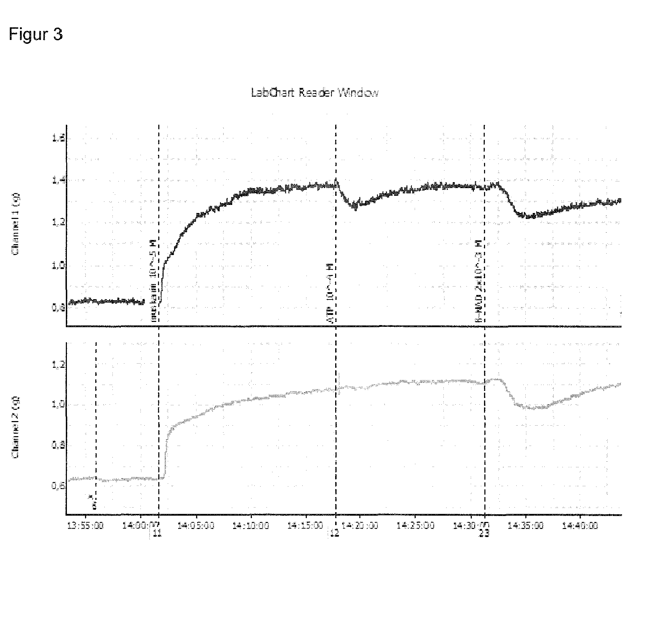

Es ist bekannt, dass ATP die purinergen Rezeptoren der P2Y-Reihe aktiviert. Für die Magen-Darm-Trakt-Muskulatur des Menschen wurde postuliert, dass β-NAD eine Dilatation dieser glatten Muskulatur bewirkt. Es existieren viele Subtypen der P2Y-Rezeptoren, deren Funktion durch unterschiedliche Hemmstoffe inhibiert werden kann. Um zu überprüfen, ob die erfindungsgemäße dilatative Wirkung von β-NAD auf die Atemwegsmuskulatur (β-NAD-Effekt) ebenfalls auf einer Beeinflussung der P2Y-Rezeptoren beruht, wurden folgende Hemmstoffe der P2Y-Rezeptoren einzeln oder zusammen eingesetzt: Suramin, PPADS und MRS2179. Exemplarisch ist in Figur 3 die simultane Gabe von allen drei Substanzen dargestellt. Die dilatative Wirkung des ATP auf die Atemwegsmuskulatur wurde erwartungsgemäß durch diese Hemmstoffe aufgehoben. Überraschenderweise wurde die dilatative Wirkung des β-NAD auf die Atemwegsmuskulatur (β-NAD-Effekt) durch diese Hemmstoffe, nämlich Suramin, PPADS und MRS2179 nicht aufgehoben, sondern die dilatative Wirkung des β-NAD auf die Atemwegsmuskulatur blieb unverändert bestehen. Hiermit wurde ausgeschlossen, dass β-NAD auf die glatten Muskelzellen der Atemwegsmuskulatur über den gleichen Signalweg wie ATP wirkt (Figur 3).

### 4. Überprüfung der Wirkung von β-NAD auf G-Protein-gekoppelte Rezeptoren (GPCR)

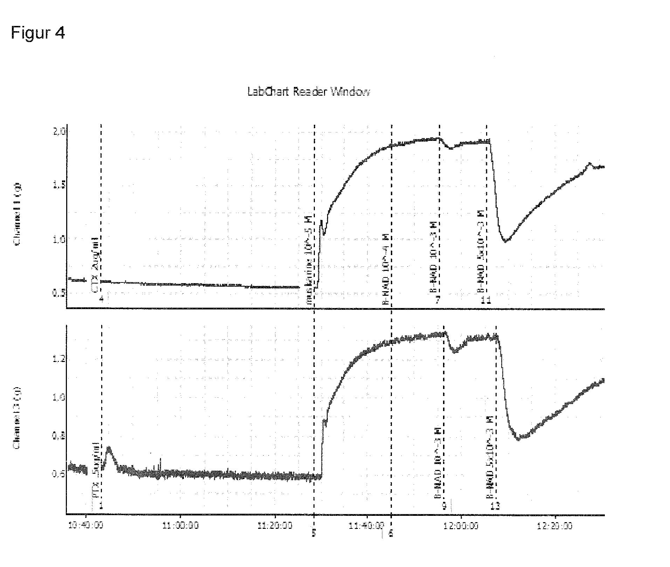

Die Wirkung von β-NAD auf G-Protein-gekoppelte Rezeptoren wurde untersucht, indem PTX und CTX eingesetzt wurden. PTX blockiert solche GPCRs, welche in der nachgeschalteten Signalkaskade inhibierend auf die Protein-Kinase A (PKA) wirken. CTX hingegen blockiert solche GPCRs, welche in der nachgeschalteten Signalkaskade aktivierend auf die Protein-Kinase A (PKA) wirken. Auch hier war keine Änderung in der dilatativen Wirkung des β-NAD auf die Atemwegsmuskulatur (β-NAD-Effekt) durch diese Hemmstoffe, nämlich PTX oder CTX zu verzeichnen. Hiermit wurde ausgeschlossen, dass β-NAD auf die glatten Muskelzellen der Atemwegsmuskulatur über den Signalweg der G-Proteingekoppelten Rezeptoren wirkt (Figur 4).

### 5. Messung der Bronchokonstriktion in lebenden Lungenschnitten der Maus durch Videomorphometrie

Hierzu wurden Veränderungen des Bronchusdurchmessers in lebenden Lungenschnitten mittels Videomorphometrie ermittelt. Nach tierschutzgerechter Tötung einer Maus wurde deren Lunge über eine in die Trachea eingeführte Kanüle mit einer 1,5%igen Agarose-Lösung gefüllt, entnommen und in eine auf 4° C gekühlte HEPES-Lösung überführt. Durch das Auskühlen der Agarose-Lösung wurde das Präparat fest, so dass durch ein Vibratom 200 µm dicke Schnitte angefertigt werden konnten. Anschließend wurden die Schnitte in MEM bei 37° C und 21% O₂ inkubiert, bis die Agarose aus den Schnitten herausgewaschen war. Die weiterhin lebenden Schnitte wurden in eine auf einer Mikroskopbühne montierten Kammer überführt und die Veränderungen im Durchmesser der Bronchi nach Zugabe verschiedener Substanzen bei 20-facher Vergrößerung beobachtet, aufgenommen und ausgewertet (Optimas Software, Saterland-Ramsloh, Deutschland).

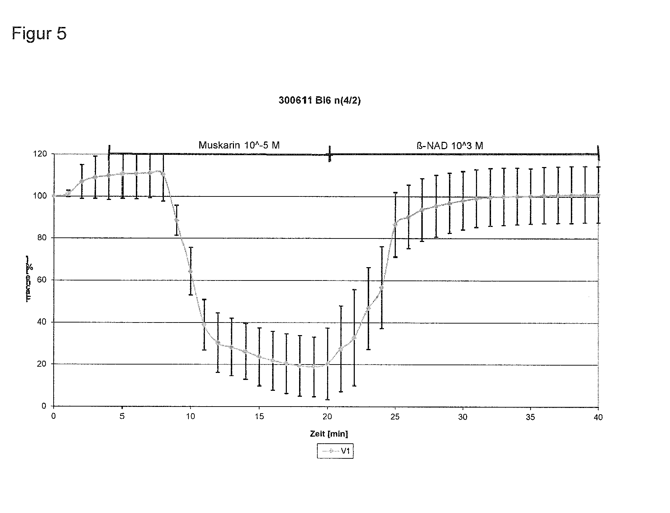

Muskarin führt in derart präparierten Bronchien zu einer Verengung der Bronchien auf ca. 20% der Ausgangsfläche. Diese Kontraktion ist in der Kurvendarstellung als Abfall im Kurvenverlauf zu sehen (Figur 5). β-NAD führt hier zu einer vollständigen Aufhebung der durch Muskarin erzielten Kontraktion, in Figur 5 als Anstieg im Kurvenverlauf zu sehen.

### Erläuterung der Figuren

Figur 1 zeigt die Dilatation der Trachealmuskulatur nach Zugabe von β-NAD, nach dem vorher durch Muskarin eine Kontraktion der Trachealmuskulatur verursacht wurde. Diese Dilatation der Trachealmuskulatur nach Zugabe von β-NAD ist abhängig von der Konzentration des β-NAD und beträgt 75-80% der maximalen durch Muskarin hervorgerufenen Kontraktion. Die x-Achse definiert den Zeitverlauf, die y-Achse die Höhe der Kontraktion der Trachealmuskulatur. Der Kurvenverlauf zeigt die Kontraktion bzw. Dilatation der Trachealmuskulatur.
Figur 2 zeigt die Dilatation der Trachealmuskulatur nach Zugabe von β-NAD, nach dem vorher durch Muskarin eine Kontraktion der Trachealmuskulatur verursacht wurde. Durch Zugabe von L-NAME kam es aber nicht zu einer Aufhebung des dilatativen Effekts durch das β-NAD (β-NAD-Effekt). Der relaxierende Effekt von β-NAD auf die Atemwegsmuskulatur besteht also auch nach Hemmung der NOS durch L-NAME. Damit wurde gezeigt, dass die dilatative Wirkung des β-NAD auf die Atemwegsmuskulatur unabhängig vom Stickstoffmonoxyd-cGMP-Signalweg ist. Die x-Achse definiert den Zeitverlauf, die y-Achse die Höhe der Kontraktion der Trachealmuskulatur. Der Kurvenverlauf zeigt die Kontraktion bzw. Dilatation der Trachealmuskulatur.
Figur 3 zeigt die Dilatation der Trachealmuskulatur nach Zugabe von β-NAD, die auch bestehen bleibt, wenn die purinergen Rezeptoren der P2Y-Reihe durch einen Inhibitorcocktail, nämlich Suramin, PPADS und MRS2179, gehemmt werden (untere Kurve). In der oberen Kurve ist zum Vergleich die dilatative Wirkung von ATP und β-NAD ohne Inhibitorcocktail dargestellt. Die x-Achse definiert den Zeitverlauf, die y-Achse die Höhe der Kontraktion der Trachealmuskulatur. Der Kurvenverlauf zeigt die Kontraktion bzw. Dilatation der Trachealmuskulatur. In diesem Versuch wurde β-NAD in einer submaximalen Dosis eingesetzt, daher ist die Dilatation nicht so ausgeprägt wie in den in den Figuren 1 und 2 gezeigten Beispielen.
Figur 4 zeigt die Dilatation der Trachealmuskulatur nach Zugabe von β-NAD, die auch bestehen bleibt, wenn der Signalweg über die G-Protein-gekoppelte Rezeptoren (GPCR) gehemmt wird. In der oberen Kurve wird GPCR durch CTX gehemmt. In der unteren Kurve wird GPCR durch PTX gehemmt. Die x-Achse definiert den Zeitverlauf, die y-Achse die Höhe der Kontraktion der Trachealmuskulatur. Der Kurvenverlauf zeigt die Kontraktion bzw. Dilatation der Trachealmuskulatur.
Figur 5 zeigt die Bronchokonstriktion nach Zugabe von Muskarin und die Bronchodilatation nach Zugabe von β-NAD in einem lebenden Lungenschnitt. Muskarin bewirkt eine Kontraktion (Verringerung der inneren Fläche der durch die Bronchialwände definierten luftdurchströmten Hohlräume der Bronchien), die durch β-NAD in einer Konzentration von 10⁻³ M aufgehoben wird. Dargestellt sind die Mittelwerte und die Standardabweichungen von 4 Bronchien aus zwei Mäusen. Die x-Achse definiert den Zeitverlauf, die y-Achse die innere Fläche. Der Kurvenverlauf zeigt die Änderung der inneren Fläche durch Zugabe von Muskarin und β-NAD.

## Patentansprüche

**4.** Mittel, umfassend NAD (Nicotinamidadenosindinukleotid) oder β-NAD oder ein pharmazeutisch annehmbares Salz, Diastereomer, Stereoisomer, Tautomer, Solvat, Polymorph, isotopische Variation davon zur Herstellung eines Medikamentes zur Behandlung und Prophylaxe von Menschen und Tieren.

**5.** Mittel nach Anspruch 1, **dadurch gekennzeichnet, dass** es zusätzlich pharmazeutisch unschädliche Exzipientien und/oder Additive umfasst.

**6.** Mittel nach Anspruch 1 oder 2, **dadurch gekennzeichnet, dass** es zusätzlich andere therapeutische Mittel aus der Gruppe der Entzündungshemmer, Bronchodilatatoren, muskarinischen Rezeptorantagonisten oder Beta-Agonisten umfasst.

**7.** Verwendung eines Mittels gemäß Anspruch 1 bis 3 zur Herstellung eines Medikamentes zur Behandlung und Prophylaxe von Erkrankungen, Störungen und Zuständen bei Menschen und Tieren, bei denen die Konzentration an freiem Kalzium in den Muskelzellen der Atemwegsmuskulatur krankhaft verändert ist.

**8.** Verwendung eines Mittels gemäß Anspruch 1 bis 3 zur Herstellung eines Medikamentes zur Behandlung und Prophylaxe von Erkrankungen, Störungen und Zuständen bei Menschen und Tieren, bei denen eine Konstriktion der Atemwegsmuskulatur vorliegt.

**9.** Verwendung eines Mittels gemäß Anspruch 1 bis 3 zur Herstellung eines Medikamentes zur Behandlung und Prophylaxe von Asthma oder chronisch obstruktiver Lungenerkrankungen (COPD).

**10.** Verwendung eines Mittels gemäß Anspruch 1 bis 3, **dadurch gekennzeichnet, dass** es als Spray, Aerosol, Trockenpulver oder Liposomen verabreicht wird.

**11.** Verwendung eines Mittels gemäß Anspruch 1 bis 3, **dadurch gekennzeichnet, dass** es inhalativ verabreicht wird.
